# FASCICULE DE BREVET EUROPEEN

(11) **EP 1 129 204 B1**
(45) Date de publication et mention de la délivrance du brevet: **01.02.2006**
(21) Numéro de dépôt: 99971861.2
(22) Date de dépôt: 09.11.1999
(51) Int. Cl.: C12N 15/861, C12N 15/63, C12N 15/53, C12N 15/11, C12N 9/02, C12N 5/10, A61K 48/00

(54) **NOUVEAU SYSTEME DE REGULATION DE L'EXPRESSION D'UN TRANSGENE**
NEUES REGULATIONSSYSTEM ZUR KONTROLLE DER EXPRESSION EINES TRANSGENS
NOVEL SYSTEM FOR REGULATING TRANSGENE EXPRESSION

(30) Priorité: 09.11.1998 FR 9814080; 03.03.1999 US 122600 P
(43) Date de publication de la demande: 05.09.2001
(73) Titulaire: Aventis Pharma S.A., 92160 Antony (FR)
(72) Inventeur: MALLET, Jacques, F-75013 Paris (FR); CORTI, Olga, F-75013 Pairs (FR)
(74) Mandataire: Gallois, Valérie
(86) Numéro de dépôt international: PCT/FR1999/002752
(87) Numéro de publication internationale: WO 2000/028062

(56) Documents cités:
- WO-A-97/20463
- WO-A-98/37185
- US-A- 5 650 298
- HU S -X ET AL: "DEVELOPMENT OF AN ADENOVIRUS VECTOR WITH TETRACYCLINE-REGULATABLE HUMAN TUMOR NECROSIS FACTOR ALPHA GENE EXPRESSION" CANCER RESEARCH, vol. 57, no. 16, 15 août 1997 (1997-08-15), pages 3339-3343, XP002068734
- CORTI O ET AL: "Intracerebral tetracycline-dependent regulation of gene expression in grafts of neural precursors" NEUROREPORT, vol. 7, no. 10, juillet 1996 (1996-07), pages 1655-1659, XP002104916 cité dans la demande
- HORELLOU P ET AL: "Direct intracerebral gene transfer of an adenoviral vector expressing tyrosine hydroxylase in a rat model of Parkinson's disease" NEUROREPORT, vol. 4, 1994, pages 49-53, XP002104828
- ADRA ET AL: "Cloning and expression of the mouse pgk-1 gene and the nucleotide sequence of its promoter" GENE, vol. 60, no. 1, 1987, pages 65-74, XP002104829 cité dans la demande
- CORTI O ET AL: "A single adenovirus vector mediates doxycycline-controlled expression of tyrosine hydroxylase in brain grafts of human neural progenitors" NATURE BIOTECHNOLOGY, vol. 17, no. 4, avril 1999 (1999-04), pages 349-354, XP002132469
- RIDET JL ET AL: "Toward autologous ex vivo gene therapy for the central nervous system with human adult astrocytes" HUM. GENE THERAP., vol. 10, no. 2, 20 janvier 1999 (1999-01-20), pages 271-280, XP002132470
- BOHL ET AL: "Control of erythropoietin delivery by doxycycline in mice after intramuscular injection of adeno-associated virus" BLOOD, vol. 92, no. 5, 1 septembre 1998 (1998-09-01), pages 1512-1517, XP002104830

### LISTE DE SEQUENCES

(1) INFORMATIONS GENERALES:
   (i) DEPOSANT:
      (A) NOM: RHONE-POULENC RORER SA
      (B) RUE: 20, avenue Raymond Aron
      (C) VILLE: ANTONY
      (E) PAYS: FRANCE
      (F) CODE POSTAL: 92165
      (H) TELECOPIE: 33155717291
   (ii) TITRE DE L' INVENTION: Nouveau systeme de regulation de l'expression d'un transgene
   (iii) NOMBRE DE SEQUENCES: 1
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 2502 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: - tTA (A)
      (B) EMPLACEMENT:1..1040
   (ix) CARACTERISTIQUE:
      (A) NOM/CLE: - UMS (B)
      (B) EMPLACEMENT:1041..2069
   (ix) CARACTERISTIQUE:
      (A) NOM/CLE: - 7 OP (C)
      (B) EMPLACEMENT:2069..2362
   (ix) CARACTERISTIQUE:
      (A) NOM/CLE: - 1 OP
      (B) EMPLACEMENT:2070..2110
   (ix) CARACTERISTIQUE:
      (A) NOM/CLE: - promoteur minimal CMV (D)
      (B) EMPLACEMENT:2363..2502
   (xi) DESCRIPTION DE LA SEQUENCE: SEQ ID NO: 1:
   CTCGAGGAGC TCGAATTCAT ATGTCTAGAT TAGATAAAAG TAAAGTGATT AACAGCGCAT 60
   TAGAGCTGCT TAATGAGGTC GGAATCGAAG GTTTAACAAC CCGTAAACTC GCCCAGAAGC 120
   TAGGTGTAGA GCAGCCTACA TTGTATTGGC ATGTAAAAAA TAAGCGGGCT TTGCTCGACG 180
   CCTTAGCCAT TGAGATGTTA GATAGGCACC ATACTCACTT TTGCCCTTTA GAAGGGGAAA 240
   GCTGGCAAGA TTTTTTACGT AATAACGCTA AAAGTTTTAG ATGTGCTTTA CTAAGTCATC 300
   GCGATGGAGC AAAAGTACAT TTAGGTACAC GGCCTACAGA AAAACAGTAT GAAACTCTCG 360
   AAAATCAATT AGCCTTTTTA TGCCAACAAG GTTTTTCACT AGAGAATGCA TTATATGCAC 420
   TCAGCGCTGT GGGGCATTTT ACTTTAGGTT GCGTATTGGA AGATCAAGAG CATCAAGTCG 480
   CTAAAGAAGA AAGGGAAACA CCTACTACTG ATAGTATGCC GCCATTATTA CGACAAGCTA 540
   TCGAATTATT TGATCACCAA GGTGCAGAGC CAGCCTTCTT ATTCGGCCTT GAATTGATCA 600
   TATGCGGATT AGAAAAACAA CTTAAATGTG AAAGTGGGTC CGCGTACAGC CGCGCGCGTA 660
   CGAAAAACAA TTACGGGTCT ACCATCGAGG GCCTGCTCGA TCTCCCGGAC GACGACGCCC 720
   CCGAAGAGGC GGGGCTGGCG GCTCCGCGCC TGTCCTTTCT CCCCGCGGGA CACACGCGCA 780
   GACTGTCGAC GGCCCCCCCG ACCGATGTCA GCCTGGGGGA CGAGCTCCAC TTAGACGGCG 840
   AGGACGTGGC GATGGCGCAT GCCGACGCGC TAGACGATTT CGATCTGGAC ATGTTGGGGG 900
   ACGGGGATTC CCCGGGTCCG GGATTTACCC CCCACGACTC CGCCCCCTAC GGCGCTCTGG 960
   ATATGGCCGA CTTCGAGTTT GAGCAGATGT TTACCGATGC CCTTGGAATT GACGAGTACG 1020
   GTGGGTAGGG GGCGCGAGGA TCTCAGATTT GTGCATACAC AGTGACTCAT ACTTTCACCA 1080
   ATACTTTGCA TTTTGGATAA ATACTAGACA ACTTTAGAAG TGAATTATTT ATGAGGTTGT 1140
   CTTAAAATTA AAAATTACAA AGTAATAAAT CACATTGTAA TGTATTTTGT GTGATACCCA 1200
   GAGGTTTAAG GCAACCTATT ACTCTTATGC TCCTGAAGTC CACAATTCAC AGTCCTGAAC 1260
   TATAATCTTA TCTTTGTGAT TGCTGAGCAA ATTTGCAGTA TAATTTCAGT GCTTTTAAAT 1320
   TTTGTCCTGC TTACTATTTT CCTTTTTTAT TTGGGTTTGA TATGCGTGCA CAGAATGGGG 1380
   CTTCTATTAA AATATTCCAT GGCTTACATT TTTAATGTTT TGTTCTCTTA ATATGTTCAA 1440
   AGCTACTCAA CTTTTATTCC CGAAAAATGT TTACTTTAAT TATTCTAATT TCTTACATAA 1500
   AGCATTGAGG TGCTAACAAT TATATACTAT GTACAAGATG GCAGACTAAA TCATATCATA 1560
   CCATCAAGTA GAAACCTGGA GTTTGGTGAA CTTTGAGTTG TTTATATGTC TCTCCTTTAT 1620
   TGTCTTCTCA AAACCTGTGA TTCTGAAGTC AAAGGGACAC AGCTGTCACA TGAAAAGTGA 1680
   TCACTTATCA CCTGTATGCG TAAAACACCT TACCAAGCAG CTAAGAGGAG TAACTCCTAG 1740
   CCACTTTGAG AAACGTTTTT GAATAAACAG AGCAAGGCTC TTCCCCATTC TCCCAGAGAT 1800
   ATAGCATAAA ACTGAGCGCA TTTTTATAAA ACAAAAAAGG AGGAATGTGT GGTTTGATGG 1860
   CCAGACCCTG AATTTGAGTT CAGCATCTGC TTTTCCATAT TATAGATGGG TACCAGTGAT 1920
   TCTGAGCCAT GTCTATTTCT CCTGACTTTT CCTCTGTTTT CCCACGCTTG CTGATATTTA 1980
   CAGCCGTGGT CATCACAATC ACCTTTGTTC CTTTCTTCCT TCCTCCAACT CTGCATTAAA 2040
   TTCCAGGAAC TTGCTTTCTG TGAAGTCTGA GTTTACCACT CCCTATCAGT GATAGAGAAA 2100
   AGTGAAAGTC GAGTTTACCA CTCCCTATCA GTGATAGAGA AAAGTGAAAG TCGAGTTTAC 2160
   CACTCCCTAT CAGTGATAGA GAAAAGTGAA AGTCGAGTTT ACCACTCCCT ATCAGTGATA   2220
   GAGAAAAGTG AAAGTCGAGT TTACCACTCC CTATCAGTGA TAGAGAAAAG TGAAAGTCGA   2280
   GTTTACCACT CCCTATCAGT GATAGAGAAA AGTGAAAGTC GAGTTTACCA CTCCCTATCA   2340
   GTGATAGAGA AAAGTGAAAG TCGAGCTCGG TACCCGGGTC GAGTAGGCGT GTACGGTGGG   2400
   AGGCCTATAT AAGCAGAGCT CGTTTAGTGA ACCGTCAGAT CGCCTGGAGA CGCCATCCAC   2460
   GCTGTTTTGA CCTCCATAGA AGACACCGGG ACCGATCCAG CC                      2502

## Description

La présente invention concerne de nouvelles compositions et méthodes pour contrôler l'expression d'acides nucléiques dans les cellules. L'invention est plus particulièrement adaptée au contrôle de l'expression d'acides nucléiques dans les cellules nerveuses, in vitro comme in vivo.

Le contrôle de l'expression des acides nucléiques dans les cellules présente un attrait majeur dans tous les domaines de la biotechnologie. Ainsi, in vitro ou ex vivo, il permet d'améliorer les conditions d'expression de protéines recombinantes, l'étude de la fonction ou la régulation de gènes, la production de particules virales, la préparation de cellules destinées à être implantées in vivo, etc. In vivo, il peut permettre d'améliorer les propriétés de modèles animaux, d'étudier plus précisément la biodisponibilité ou la toxicité de composés ou la fonction des gènes, ou encore, de contrôler plus efficacement la production de composés dans un but thérapeutique ou vaccinal, par exemple. Les compositions et méthodes de l'invention sont utilisables dans tous ces domaines de la biotechnologie.

Différentes approches ont été décrites dans l'art antérieur pour tenter de contrôler l'expression de gènes. Il s'agit en particulier de l'utilisation de vecteurs ou promoteurs transcriptionnels présentant une spécificité tissulaire. Néanmoins, les systèmes décrits ne présentent pas toujours les niveaux de spécificité attendus, notamment in vivo, et ils ne permettent pas un contrôle temporel de l'expression.

Un système complexe, utilisant d'une part le transactivateur tTA contrôlé par la tétracycline et d'autre part un promoteur tTA sensible a été décrit par Gossen et al. (PNAS 89 (1992) 5547 ; Science 268 (1995) 1766). Bien qu'offrant des avantages en terme de régulation temporelle de l'expression, ce système, tel que décrit jusqu'à présent, présente toujours certains inconvénients qui limitent ses applications. Ces inconvénients sont notamment la toxicité cellulaire, les fuites d'expression observées, la nécessité d'utiliser deux vecteurs, etc.

Hu et al, 1997, Cancer Research 57, 3339-3343, décrit un système d'expression régulé par la tétracycline mettant en oeuvre un promoteur CMV modifié.

US 5 650 298 décrit des constructions de ciblage permettant d'introduire par recombinaison homologue le transgène codant pour le transactivateur tTA.

La présente invention offre à présent un nouveau système d'expression régulé ayant des propriétés de stabilité, spécificité et maniabilité améliorées par rapport aux systèmes de l'art antérieur.

Un premier aspect de l'invention est donc relatif à des constructions géniques comprenant des éléments, dont la nature et l'agencement particuliers, permettent d'assurer un contrôle étroit de l'expression génique.

Un autre aspect de l'invention concerne des vecteurs incorporant ces constructions génétiques, et en particulier des vecteurs viraux.

Un autre aspect de l'invention est donc relatif à des cellules nerveuses génétiquement modifiées par les constructions génétiques ou vecteurs de l'invention, des composition les comprenant, et leur utilisation pour la production de produits d'intérêt in vitro, ex vivo ou in vivo.

Un autre aspect de l'invention concerne des méthodes et compositions pour l'expression régulée d'un acide nucléique dans les cellules in vitro, ex vivo ou in vivo, .plus particulièrement dans les cellules nerveuses. Un autre aspect de l'invention est encore relatif à une méthode pour le transfert et l'expression régulée d'un acide nucléique in vivo comprenant l'implantation (ou la greffe) in vivo de cellules (notamment de progéniteurs nerveux) modifiées génétiquement par des constructions ou vecteurs de l'invention.

La présente invention décrit plus particulièrement un nouveau système d'expression régulé, basé sur l'utilisation de tTA, et ayant des propriétés améliorées. Ce système est particulièrement efficace dans un contexte adénoviral, et permet l'expression efficace et régulée in vitro comme in vivo, tout particulièrement dans le système nerveux.

Un premier objet de l'invention concerne plus particulièrement un acide nucléique caractérisé en ce qu'il comprend :
a) une première région comprenant un acide nucléique codant pour le transactivateur tTA du système de régulation par la tétracycline, sous contrôle d'un promoteur non-viral modéré, et,
b) une deuxième région comprenant un acide nucléique d'intérêt sous contrôle d'un promoteur tTA sensible,

et en ce que les deux régions a) et b) sont disposées dans la même orientation transcriptionnelle.

Plus préférentiellement, l'acide nucléique selon l'invention comprend, en outre, une troisième région c), disposée entre les deux régions a) et b), limitant les interférences transcriptionnelles entre les régions a) et b).

La présente invention résulte en effet de la mise en évidence des propriétés avantageuses de telles constructions, notamment pour le contrôle de l'expression génique in vivo, en particulier dans les cellules nerveuses. L'invention montre également que les propriétés avantageuses de ces constructions résultent, en partie, dans le choix et l'agencement des différents éléments génétiques entre eux.

Ainsi, dans la région a) des acides nucléiques de l'invention, le promoteur utilisé pour contrôler l'expression du gène transactivateur tTA est un promoteur non-viral modéré. Le terme "promoteur modéré" désigne au sens de l'invention un promoteur dont le niveau d'activité est considéré comme moyen par l'homme du métier, c'est-à-dire inférieur à celui des promoteurs forts. Plus particulièrement, un promoteur modéré au sens de l'invention est un promoteur non-viral permettant une expression à des niveaux physiologiques.

Ainsi, contrairement aux systèmes antérieurement décrits (Gossen et al., PNAS 89 (1992) 5547) dans lesquels le promoteur utilisé est un promoteur viral fort (hCMV notamment), les constructions de l'invention permettent la synthèse constitutive du transactivateur tTA, à des niveaux non toxiques pour les cellules mammifères. La demanderesse a maintenant montré que le maintien de niveaux modérés de tTA dans les cellules permet d'obtenir une régulation beaucoup plus fine et précise de l'expression génique, notamment dans les cellules nerveuses.

Plus préférentiellement, le promoteur utilisé est un promoteur cellulaire, c'est-à-dire d'un promoteur provenant d'un gène exprimé dans une cellule. Avantageusement, il s'agit d'un promoteur provenant (ou dérivé) d'une cellule issue d'un organisme de la même espèce que les cellules dans lesquelles la construction génétique est destinée à être introduite. Ainsi, lorsque les constructions de l'invention sont destinées à l'introduction d'acides nucléiques chez des animaux, le promoteur cellulaire modéré utilisé est préférentiellement dérivé d'une cellule d'un animal de la même espèce, ou d'une espèce apparentée ou proche. Bien entendu, il est possible d'utiliser des promoteurs dérivés d'organismes différents, par exemple un promoteur murin pour l'expression chez l'homme, dès lors que le promoteur utilisé est fonctionnel.

Encore plus préférentiellement, le promoteur utilisé est un promoteur cellulaire modéré constitutif. La présente demande montre en effet que les meilleurs propriétés du système de l'invention sont obtenues lorsque le promoteur utilisé est constitutif, c'est-à-dire assure la présence et le maintien de niveaux stables de tTA dans les cellules.

Des exemples particuliers de promoteurs cellulaires modérés constitutifs adaptés à la présente invention sont notamment des promoteurs ubiquitaires tels que le promoteur du gène domestique ("housekeeping") de la 3-phosphoglycérate kinase (PGK), de la dihydrofolate réductase (DHFR) ou encore du facteur d'élongation 1α (EF1α). D'autres promoteurs cellulaires modérés utilisables dans le cadre de l'invention sont des promoteurs spécifiques de tissus, tels que par exemple le promoteur des gènes GFAP ("Glial Specific Fibrillary Acidic Protein"), spécifique de la glie, NSE ("Neuron Specific Enolase"), spécifique des neurones, β-actine, β-globine'. MHCα ("Myosis Heavy Chain α"), spécifique du muscle cardiaque, ou encore des promoteurs composés, par exemple de type NRSE-PGK.

Des promoteurs modérés au sens de l'invention sont donc avantageusement des promoteurs cellulaires, assurant une expression constitutive, ubiquitaire ou localisée à certains tissus ou types cellulaires, permettant de préférence d'obtenir des niveaux physiologiques d'expression. L'utilisation du promoteur PGK (ou de variants) représente un mode de mise en oeuvre préféré de l'invention. Ainsi, les exemples présentés plus loin montrent que l'utilisation de ce promoteur permet de maintenir in vivo l'expression de l'acide nucléique d'intérêt au moins un mois après l'introduction de la construction. Ainsi, aucune diminution du nombre de cellules exprimant l'acide nucléique d'intérêt n'a été observée au cours de cette période. Cette stabilité d'expression représente un des avantages importants de la présente invention en comparaison avec les systèmes de l'art antérieur. Ainsi, dans des greffes de cellules nerveuses progénitrices infectées avec un adénovirus codant pour la b-galactosidase sous le contrôle d'un promoteur fort (promoteur du LTR du virus RSV), une diminution claire des niveaux d'expression est observée moins d'un mois après la greffe. Ceci illustre donc les propriétés avantageuses de l'invention, en terme de stabilité, liées, de manière inattendue, à l'emploi d'un promoteur modéré pour diriger l'expression du gène tTA.

Une autre caractéristique importante des constructions selon l'invention réside dans l'agencement des deux régions a) et b). Ainsi, ces deux régions sont avantageusement (i) disposées dans la même orientation transcriptionnelle et (ii) séparées par une région c) limitant les interférences transcriptionnelles.

La présente demande montre tout d'abord que, pour obtenir une meilleure efficacité du système de l'invention (c'est-à-dire un meilleur contrôle de l'expression), il est particulièrement avantageux de positionner les deux cassettes a) et b) dans la même orientation transcriptionnelle. Ces résultats sont plutôt surprenants dans la mesure où un meilleur contrôle de l'expression a souvent été observé lorsque deux cassettes sont positionnées en orientation inverse. Dans le cadre de la présente invention, au contraire, aucune fuite ne semble se produire, même dans un contexte adénoviral, lorsque les cassette sont dans la même orientation, alors que de telles fuites avaient été rapportées pour une construction en orientation anti-parallèle (Kojima et al., Biochem.Biophys.Res.Commun 238 (1997) 569).

D'autre part, pour améliorer encore les propriétés des constructions de l'invention, il est particulièrement avantageux d'introduire, entre les régions a) et b) mentionnées ci-dessus, une troisième région c) limitant les interférences transcriptionnelles entre a) et b). L'expression "interférence transcriptionnelle" désigne toute influence ou effet du promoteur de la région a) sur la transcription de l'acide nucléique b), et réciproquement. Plus spécifiquement, on désigne par "interférence transcriptionnelle" la différence pouvant exister entre l'expression de la région b) d'une part dans un contexte isolé et d'autre part liée de manière fonctionnelle à la région a). Une telle région c) comprend préférentiellement un terminateur transcriptionnel, de préférence la séquence UMS. La séquence UMS ("Upstream Mouse Sequence") est une séquence génomique, qui a été identifiée en amont du gène c-mos murin (McGeady et al., Dna 5 (1986) 289). Cette séquence se comporte comme un terminateur transcriptionnel. La présente invention montre maintenant que ces séquences peuvent être utilisées avantageusement pour le contrôle efficace de l'expression de gènes in vivo, en particulier dans le système nerveux, plus spécifiquement dans un contexte adénoviral.

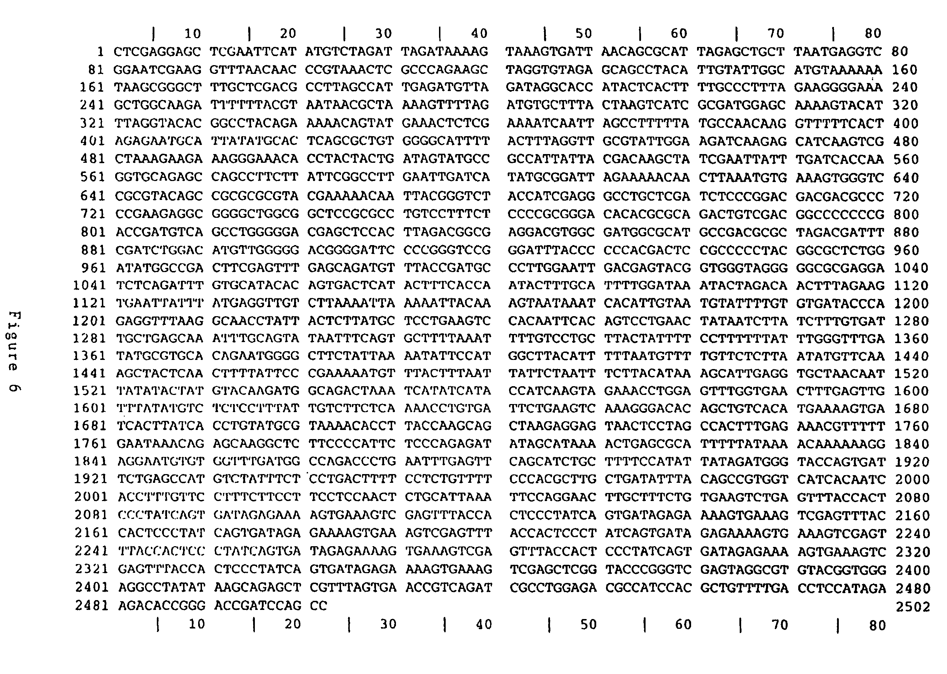

Le système de l'invention repose donc sur la présence, dans un même acide nucléique, de deux cassettes (régions), l'une exprimant, dans des conditions régulables, un facteur transcriptionnel capable d'activer l'expression, dans la seconde cassette d'un acide nucléique d'intérêt. Dans cette seconde cassette (la région b)), il est donc important de choisir un promoteur transcriptionnel qui soit adapté à la construction génique de l'invention. Ainsi, dans la région b), le promoteur utilisé est un promoteur sensible au transactivateur tTA, c'est-à-dire dont l'activité est augmentée en présence dudit transactivateur. D'un point de vue structural, il s'agit donc d'un promoteur comprenant, dans sa séquence ou à une distance fonctionnelle de celle-ci, un site au moins de liaison du facteur tTA (Gossen et al., 1992). Ce site de liaison, ou région opératrice (Op ou tetOp) peut posséder par exemple la séquence représentée sur la figure 6 ou un variant fonctionnel de celle-ci. Un variant peut correspondre par exemple à une séquence modifiée génétiquement, par mutation, déletion ou ajout d'une ou plusieurs paires de bases, et conservant la capacité de lier le transactivateur tTA. Préférentiellement, les modifications concernent moins de 10% de la séquence présentée sur la figure 6. En outre, la séquence Op peut être présente, dans la région b), en une ou plusieurs copies, par exemple de 1 à 10 copies, préférentiellement de 3 à 10, encore plus préférentiellement de 3 à 8 copies. Dans un mode particulier de mise en oeuvre, le promoteur utilisé en b) comporte 7 séquences opératrices. Encore plus préférentiellement; afin d'assurer un contrôle important de l'expression de l'acide nucléique d'intérêt, il est fortement recommandé que le promoteur utilisé en b) ait une activité basale très faible, voire nulle. De ce fait, une activité de ce promoteur n'est observée qu'en présence du tTA, et la régulation est donc très étroite. Avantageusement, le promoteur utilisé en b) est donc un promoteur minimal sensible au transactivateur tTA. La caractère minimal désigne un promoteur dont l'activité basale est très faible, voire nulle. Un tel promoteur comprend généralement les éléments minimum indispensables à la fonction de promoteur transcriptionnel (boite TATA par exemple) et est dépourvu des autres régions naturellement impliquées dans la force du promoteur ou sa régulation, par exemple. De tels promoteurs peuvent être préparés à partir de différents types de promoteurs, par délétion et/ou ajout d'éléments ("silencer"). Un promoteur préféré en b) est un promoteur fonctionnel dans les cellules mammifères, essentiellement inactif en l'absence de tTA, et stimulé en présence du transactivateur tTA. Le terme "essentiellement inactif" désigne l'absence de produit d'expression (polypeptide) détectable par les techniques classiques, et notamment par dosage enzymatique ou par des techniques d'immunohistochimie, de microdyalyse ou encore de HPLC. Ce terme n'exclut pas la présence d'ARNm détectables par PCR ou autres techniques très sensibles de détection, mais dont le niveau de concentration demeure pratiquement non significatif.

Le promoteur utilisé en b) peut être de nature, d'origine et avoir des propriétés variées. Le choix du promoteur utilisé dépend en effet de l'utilisation recherchée et du gène d'intérêt, notamment. Ainsi, le promoteur peut être issu d'un promoteur fort ou faible, ubiquitaire ou spécifique de tissus/cellules, ou encore spécifique d'états physiologiques ou physiopathologiques (activité dépendante de l'état de différenciation cellulaire ou de certaines étapes du cycle cellulaire), par exemple. Le promoteur peut être d'origine eucaryote, procaryote, virale, animale, végétale, artificielle ou humaine, etc. Des exemples particuliers de promoteurs sont le promoteur des gènes précoces immédiats CMV, TK, GH, EF1-α, APO, etc. ou des promoteurs artificiels. Pour leur utilisation dans la présente invention, ces promoteurs sont de préférence rendus "minimum", c'est-à-dire dépendants de la présence du tTA. Pour cela, les promoteurs d'origine peuvent être digérés par des enzymes et testés pour leur activité, de préférence après couplage fonctionnel avec une ou plusieurs séquences Op. Le nombre de séquences tetOp, ainsi que leur distance par rapport au promoteur choisi, peuvent être adaptés par l'homme du métier de façon à ce que l'expression dudit promoteur soit strictement tTA dépendante. Préférentiellement, le promoteur utilisé en b) est un promoteur minimal du gène précoce immédiat du cytomégalovirus (CMV), de préférence du CMV humain (hCMV), tel que décrit dans Corti et al (Neuroreport 7 (1996) 1655).

La présente invention peut être utilisée pour l'expression d'acides nucléiques d'intérêt variés, selon les applications recherchées. Ainsi, dans la région b), l'acide nucléique d'intérêt est avantageusement un acide nucléique codant pour une protéine ou un polypeptide d'intérêt. Parmi les produits d'intérêt au sens de la présente invention, on peut citer plus particulièrement les enzymes, les dérivés sanguins, les hormones telles que l'hormone de croissance, les cytokines, les lymphokines : interleukines, interférons, TNF, etc. (Brevet français n° 92 03120), les facteurs de croissance, par exemples les facteurs angiogéniques tels que les VEGF ou FGF, les neurotransmetteurs ou leurs précurseurs ou enzymes de synthèse (Tyrosine hydroxylase: TH), les facteurs trophiques, en particulier neurotrophiques pour le traitement des maladies neurodégénératives, des traumatismes ayant endommagé le système nerveux, ou des dégénérescences rétiniennes : BDNF, CNTF, NGF, IGF, GMF aFGF, NT3. NT5, HARP/pléiotrophine, ou les facteurs de croissance osseuse, les facteurs hématopoïétiques, etc., la dystrophine ou une minidystrophine (brevet français n° 91 11947), les gènes codant pour des facteurs impliqués dans la coagulation : facteurs VII, VIII, IX, les gènes suicides (thymidine kinase, cytosine déaminase), les protéines impliquées dans le cycle cellulaire comme p21, ou autres protéines inhibitrices des kinases dépendantes, Rb, Gas-1, Gas-6, Gas-3, Gad 45, Gad 153, les cyclines A, B, D, ou encore la protéine GAX limitant la prolifération des cellules dans les muscles lisses (traitement de la resténose), les protéines induisant l'apoptose ou d'autres suppresseurs de tumeurs comme p53, Bax, BcIX-s, Bad ou tout autre antagoniste de Bcl2 et de BcIX-1, les gènes de l'hémoglobine ou d'autres transporteurs protéiques, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple la lipoprotéine lipase, la lipase hépatique, la lécithine cholestérol acyltransférase, la 7 alpha cholestérol hydroxylase, la phosphatidyl acide phosphatase, ou encore des protéines de transfert de lipides comme la protéine de transfert des esters de cholestérol et la protéine de transfert des phospholipides, une protéine de liaison des HDL ou encore un récepteur choisi parmi les récepteurs aux LDL, les récepteurs des chylomicrons-remnants et les récepteurs scavenger, etc.

Parmi les produits d'intérêt il est important de souligner les anticorps, les fragments variables d'anticorps simple chaîne (ScFv) ou tout autre fragment d'anticorps possédant des capacités de reconnaissance pour son utilisation en immunothérapie, par exemple pour le traitement des maladies infectieuses, des tumeurs (anticorps anti-RAS, anti-p53 ou anti-GAP), des maladies autoimmunes telles que la sclérose en plaques (anticorps antiidiotype).

D'autres protéines d'intérêt sont, de façon non limitative, des récepteurs solubles, comme par exemple le récepteur CD4 soluble ou le récepteur soluble du TNF pour la thérapie anti-HIV, le récepteur soluble de l'acétylcholine pour le traitement de la myasthénie ; des peptides substrats ou inhibiteurs d'enzymes, ou bien des peptides agonistes ou antagonistes de récepteurs ou de protéines d'adhésion comme par exemple pour le traitement de l'asthme, de la thrombose et de la resténose : des protéines artificielles, chimériques ou tronquées. Parmi les hormones d'intérêt essentiel, on peut citer l'insuline dans le cas du diabète, l'hormone de croissance et la calcitonine.

L'acide nucléique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaire d'ARNm cellulaires et bloquer ainsi leur traduction en protéine, selon la technique décrite dans le brevet européen n° 140 308. Les gènes thérapeutiques comprennent également les séquences codant pour des ribozymes, qui sont capables de détruire sélectivement des ARN cibles (brevet européen n° 321 201).

L'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins, soit de traitements immunothérapeutiques appliqués à l'homme ou à l'animal, notamment contre des microorganismes, des virus ou des cancers. Il peut s'agir notamment de peptides antigèniques spécifiques du virus d'Epstein Barr, du virus HIV, du virus de l'hépatite B (brevet Européen N° 185 573), du virus de la pseudo-rage, du "syncitia forming virus", d'autres virus ou encore d'antigènes spécifiques de tumeurs comme les protéines MAGE (brevet Européen n° 259 212).

L'acide nucléique peut également coder pour un produit toxique pour les cellules, notamment une toxicité conditionnelle (i.e., thymidine kinase, cytosine désaminase, etc.).

D'autres gènes présentant un intérêt ont été notamment décrits par Mc Kusick, V.A. Mendelian (Inheritance in man, catalogs of autosomal dominant, autosomal recessive, and X-linked phenotypes, Eighth edition. John Hopkins University Press (1988)), et dans Standbury, J. B. et al. (The metabolic basis of inherited disease, Fifth edition. McGraw-Hill (1983)). Les gènes d'intérêt recouvrent les protéines impliquées dans le métabolisme des acides aminés, des lipides et des autres constituants de la cellule.

D'autre part, la région b) peut également comprendre, en 3' de l'acide nucléique d'intérêt, un terminateur transcriptionnel. Enfin, l'acide nucléique peut comporter plusieurs régions codantes, éventuellement séparées par une IRES, permettant la production de plusieurs produits d'intérêt.

Les constructions selon l'invention étant particulièrement adaptées à la régulation de l'expression dans le système nerveux, les acides nucléiques d'intérêt codent plus particulièrement des neurotransmetteurs ou leurs précurseurs ou enzymes de synthèse (Tyrosine hydroxylase: TH), des facteurs trophiques, en particulier neurotrophiques pour le traitement des maladies neurodégénératives, des traumatismes ayant endommagé le système nerveux, ou des dégénérescences rétiniennes.

Dans un mode de réalisation particulier, l'invention concerne un acide nucléique caractérisé en ce qu'il comprend :
a) une première région comprenant un acide nucléique codant pour le transactivateur du système de régulation par la tétracycline (tTA) sous contrôle du promoteur du gène PGK, et,
b) une deuxième région comprenant un acide nucléique d'intérêt, notamment codant pour la tyrosine hydroxylase humaine, sous contrôle du promoteur CMV minimal modifié de façon à inclure 1 à 10, de préférence 3 à 8, notamment 7 séquences tetOp.
c) une troisième région comprenant une séquence UMS,

et en ce que les deux régions a) et b) sont disposées dans la même orientation transcriptionnelle.

L'acide nucléique de l'invention peut être de l'ADN comme de l'ARN. Il peut s'agir plus particulièrement d'un ADN génomique (ADNg) ou complémentaire (ADNc). Les acides nucléiques de l'invention peuvent être préparés selon les techniques bien connues de l'homme du métier, impliquant par exemple la synthèse d'acides nucléiques, le criblage de banques, la ligation, les coupures enzymatiques, l'amplification, le clonage, etc. comme illustré dans les exemples (voir Maniatis et al.). Ainsi, les différentes régions a), b) et c) peuvent être préparées séparément, puis assemblées de manière fonctionnelle, c'est-à-dire dans la même orientation transcriptionnelle. L'espace entre les régions a) et b) peut être variable sans que cela nuise à l'efficacité des constructions de l'invention. Ainsi, ces régions peuvent être séparées de 0 à 3000 pb par exemple, plus généralement de 0 à 1000 pb, avantageusement de moins de 500 bp. Cette distance entre les régions a) et b) est généralement déterminée par la longueur de la région c) (lorsqu'elle est présente) et par la capacité de clonage du vecteur utilisé. Elle peut être aisément adaptée par l'homme du métier. Les acides nucléiques de l'invention peuvent comprendre des éléments d'origine variée, notamment provenant d'acides nucléiques procaryote, eucaryote (animal, végétal, viral, etc.), synthétique ou semi-synthétique.

La présente invention a également pour objet tout vecteur comprenant un acide nucléique tel que défini ci-avant. Il peut s'agir de tout vecteur connu de l'homme du métier, tel que par exemple un plasmide, cosmide, phage, YAC, HAC, transposon, épisome, virus, etc. Un type de vecteurs préféré selon l'invention est représenté par les vecteurs viraux, tels que par exemple les adénovirus, les AAV, virus de l'herpès, de la vaccine, ou encore certains rétrovirus (notamment les lentivirus). Il s'agit encore plus préférentiellement d'un virus ayant un tropisme pour les cellules nerveuses, notamment les progéniteurs de cellules nerveuses. A cet égard, un vecteur particulièrement préféré dans le cadre de la présente invention est un adénovirus.

Les adénovirus utilisés pour la mise en oeuvre de la présente invention sont avantageusement des adénovirus recombinants défectifs, c'est-à-dire dont le génome comprend un acide nucléique hétérologue et qui sont incapables de réplication autonome dans les cellules. Plus préférentiellement, les adénovirus de l'invention sont défectifs pour tout ou partie des régions E1 et E3 au moins.

Il peut s'agir également d'adénovirus recombinants défectifs de 3ème génération, c'est-à-dire défectifs pour les régions E1 et E4, en tout ou en partie, et éventuellement pour la région E3.

Des variantes particulières de l'invention sont constituées par l'utilisation d'adénovirus portant des délétions affectant tout ou une partie fonctionnelle des régions suivantes :
- E1, E4 et E3,
- E1, E4 et E2,
- E1, E4, E2 et E3,
- les régions ci-dessus ainsi que tout ou partie des gènes codant pour les fonctions tardives de l'adénovirus (L1 à L5), ou encore,
- l'ensemble des régions virales codantes.

La structure génomique des adénovirus a été largement décrite dans la littérature. A cet égard, le génome de l'adénovirus Ad5 a été entièrement séquencé et est accessible sur base de données (voir notamment GeneBandk M73260). De même, des parties, voire la totalité d'autres génomes adénoviraux (Ad2, Ad7, Ad12, adénovirus canin CAV-2, etc.) ont également été séquencées. De plus, la construction d'adénovirus recombinants défectifs a aussi été décrite dans la littérature. Ainsi, les demandes WO 94/28152, WO 95/02697 et WO 96/22378 par exemple, décrivent différentes délétions dans les régions E1 et E4. De même, la demande WO 96/10088 décrit des vecteurs portant une modification au niveau du gène Iva2, la demande WO 94/26914 décrit des adénovirus d'origine animale, la demande WO 95/29993 décrit des délétions portant sur la région E2 de l'adénovirus.

Avantageusement, l'adénovirus recombinant utilisé dans le cadre de l'invention comprend une délétion dans la région E1 de son génome affectant les régions E1a et E1b. A titre d'exemple précis, on peut citer des délétions affectant les nucléotides 454-3328; 382-3446 ou 357-4020 (par référence au génome de l'Ad5).

D'autre part, la délétion dans la région E4 affecte préférentiellement l'ensemble des phases ouvertes, telles que par exemple les délétions 33466-35535 ou 33093-35535 ou une partie seulement de la région E4 (ORF6 ou ORF3 par exemple), comme décrit dans les demandes WO95/02697 et WO96/22378.

Concernant les adénovirus dépourvus en plus des fonctions tardives (vecteur "minimum") ou de l'ensemble des régions codantes (vecteur "gutless"), leur construction a été décrite par exemple par Parks et al. PNAS 93 (1996) p. 13565 et Lieber et al., J. Virol. 70 (1996) p. 8944.

Les acides nucléiques de l'invention peuvent être insérés en différents sites du génome adénoviral recombinant. Ils peuvent être insérés au niveau de la région E1, E3 ou E4, en remplacement des séquences délétées ou en surplus. Ils peuvent également être insérés en tout autre site, en dehors des séquences nécessaires en cis à la production des virus (séquences ITR et séquence d'encapsidation).

Par ailleurs, les adénovirus recombinants peuvent être d'origine humaine ou animale. Concernant les adénovirus d'origine humaine, on peut citer préférentiellement ceux classés dans le groupe C, en particulier les adénovirus de type 2 (Ad2), 5 (Ad5) ; ou les adénovirus 7 (Ad7) ou 12 (Ad12). Parmi les différents adénovirus d'origine animale, on peut citer préférentiellement les adénovirus d'origine canine, et notamment toutes les souches des adénovirus CAV2 [souche manhattan ou A26/61 (ATCC VR-800) par exemple]. D'autres adénovirus d'origine animale sont cités notamment dans la demande WO94/26914.

Les adénovirus recombinants sont produits dans une lignée d'encapsidation, c'est-à-dire une lignée de cellules capables de complémenter en trans une ou plusieurs des fonctions déficientes dans le génome adénoviral recombinant. Parmi les lignées d'encapsidation connues par l'homme du métier, on peut citer par exemple la lignée 293 dans laquelle une partie du génome de l'adénovirus a été intégrée. Plus précisément, la lignée 293 est une lignée de cellules embryonnaires humaines de rein contenant l'extrémité gauche (environ 11-12 %) du génome de l'adénovirus sérotype 5 (Ad5), comprenant l'ITR gauche, la région d'encapsidation, la région E1, incluant E1a et E1b, la région codant pour la protéine pIX et une partie de la région codant pour la protéine plVa2. Cette lignée est capable de trans-complémenter des adénovirus recombinants défectifs pour la région E1, c'est-à-dire dépourvus de tout ou partie de la région E1, et de produire des stocks viraux ayant des titres élevés. Cette lignée est également capable de produire, à température permissive (32°C), des stocks de virus comportant en outre la mutation E2 thermosensible. D'autres lignées cellulaires capables de complémenter la région E1 ont été décrites, basées notamment sur des cellules de carcinome de poumon humain A549 (WO94/28152) ou sur des rétinoblastes humains (Hum. Gen. Ther. (1996) 215). Par ailleurs, des lignées capables de trans-complémenter plusieurs fonctions de l'adénovirus ont également été décrites. En particulier, on peut citer des lignées complémentant les régions E1 et E4 (Yeh et al., J. Virol. Vol. 70 (1996) pp 559-565; Cancer Gen. Ther. 2 (1995) 322 ; Krougliak et al., Hum. Gen. Ther. 6 (1995) 1575) et des lignées complémentant les régions E1 et E2 (WO94/28152, WO95/02697, WO95/27071) ou des lignées dérivées de celles-ci utilisables pour produire des adénovirus minimum, en particulier parce qu'elles expriment en outre une activité de recombinase site-spécifique impliquée dans la construction de tels virus.

Les adénovirus recombinants sont habituellement produits par introduction de l'ADN viral dans la lignée d'encapsidation, suivie d'une lyse des cellules après environ 2 ou 3 jours (la cinétique du cycle adénoviral étant de 24 à 36 heures). Pour la mise en oeuvre du procédé, l'ADN viral introduit peut être le génome viral recombinant complet, éventuellement construit dans une bactérie (WO96/25506) ou dans une levure (WO95/03400), transfecté dans les cellules. Il peut également s'agir d'un virus recombinant utilisé pour infecter la lignée d'encapsidation. L'ADN viral peut aussi être introduit sous forme de fragments portant chacun une partie du génome viral recombinant et une zone d'homologie permettant, après introduction dans la cellule d'encapsidation, de reconstituer le génome viral recombinant par recombinaison homologue entre les différents fragments.

Après la lyse des cellules, les particules virales recombinantes peuvent être isolées par toute technique connue telle que la centrifugation en gradient de chlorure de césium ou la chromatographie. Une méthode alternative a été notamment décrite dans la demande FR 9608164.

Un autre objet de l'invention concerne une composition comprenant un vecteur tel que défini ci-avant, de préférence un adénovirus recombinant défectif tel que défini ci-avant. Dans ce mode de réalisation, les compositions selon l'invention peuvent comprendre des quantités variables d'adénovirus recombinants, aisément adaptables par l'homme du métier en fonction des applications envisagées (in vitro, ex vivo ou in vivo, par exemple). Généralement, les compositions comprennent de l'ordre de 10⁵ à 10¹⁵ p.v. d'adénovirus recombinant, de préférence de 10⁷ à 10¹² p.v. Le terme p.v. correspond au nombre de particules virales présentes dans les compositions.

Les vecteurs et compositions de l'invention peuvent être utilisés soit directement in vitro ou in vivo pour le transfert et l'expression régulée d'acides nucléiques dans les cellules, soit encore pour l'introduction d'acides nucléiques dans les cellules en vue de leur implantation (ou greffe) in vivo.

Un autre objet de l'invention concerne toute cellule nerveuse comprenant un acide nucléique ou un vecteur tels que définis ci-avant. Préférentiellement, il s'agit d'une cellule mammifère, de préférence humaine. Dans un mode particulièrement préféré, il s'agit d'une cellule cellule nerveuse progénitrice. Un telle population de cellules est par exemple la cellule progénitrice nerveuse humaine telle que décrite par Buc et al. (Neurobiol.Dis. 2 (1995) 37).

A cet égard, un mode de réalisation particulier de l'invention comprend une cellule nerveuse génétiquement modifiée par un adénovirus recombinant comprenant un acide nucléique tel que défini ci-avant.

L'invention concerne également toute composition comprenant des cellules nerveuses telles que décrites ci-dessus.

L'invention concerne encore l'utilisation d'un acide nucléique ou d'un vecteur ou d'une composition tels que décrits ci-avant, pour la préparation d'une çomposition destinée au traitement de maladies neurodégénératives, des traumatismes ayant endommagés le système nerveux ou des dégénérescences rétiniennes. De préférence, la maladie dégénérative est la maladie de Parkinson.

Dans un mode particulier, l'invention décrit une méthode pour l'expression régulée d'acides nucléiques dans le système nerveux comprenant l'implantation, dans le système nerveux d'un sujet, d'une cellule telle que définie ci-dessus, et le contrôle de l'expression par administration au sujet de tétracycline ou d'un analogue de tétracycline.

Les compositions de l'invention peuvent se présenter sous différentes formes, telles que solutions, gels, poudre, etc. Elles sont généralement sous forme de solutions, préférentiellement stériles, telles que par exemple des solutions salines (phosphate monosodique, disodique, chlorure de sodium, potassium, calcium ou magnésium, etc, ou des mélanges de tels sels), isotoniques, ou de compositions sèches, notamment lyophilisées, qui, par addition selon le cas d'eau stérilisée ou de sérum physiologique, permettent la constitution de solutés. D'autres excipients peuvent être utilisés tels que par exemple un hydrogel. Cet hydrogel peut être préparé à partir de tout polymère (homo ou hétéro) biocompatible et non cytotoxique. De tels polymères ont par exemple été décrits dans la demande WO93/08845. En outre, les compositions selon l'invention peuvent être conditionnées dans tout type de dispositif adapté, tel que flacon, tube, ampoule, poche, seringue, ballonet, etc.

Comme indiqué ci-avant, les compositions de l'invention peuvent être utilisées in vitro, ex vivo ou in vivo.

Pour une utilisation in vitro ou ex vivo, les cellules peuvent être simplement incubées, dans tout dispositif approprié (plaque, boîte, poche, etc.) en présence d'une composition de vecteur telle que décrite ci-avant. Pour ce type d'application, les compositions comprenant des adénovirus peuvent être utilisées à des multiplicités d'infection (MOI) comprises entre 10 et 5000 p.v. par cellule, par exemple, de préférence entre 100 et 2000.

Pour une utilisation in vivo, les compositions de l'invention peuvent être formulées en vue d'une administration par voie topique, orale, parentérale, intranasale, intraveineuse, intramusculaire, sous-cutanée, intraoculaire, transdermique, intratumorale, etc. Dans cette application, notamment pour des compositions cellulaires, les compositions utilisées comprennent avantageusement de 10⁴ à 10⁶ cellules, plus préférentiellement de 10⁵ à 10⁶ cellules. En outre, les compositions implantées comprennent préférentiellement des cellules en densité élevée (environ 10⁵ à 10⁶ par µl). Les résultats présentés dans les exemples montrent en effet que l'utilisation de cellules à haute densité favorise la prise de la greffe et la survie des cellules greffées. D'autre part, pour favoriser encore plus la survie des greffes in vivo, il est également possible d'administrer, de manière simultanée ou préalable (voire postérieure), un ou plusieurs composés/traitement immunosuppresseur (Neuroscience 78 (1997) 685).

A cet égard, l'invention décrit également une méthode pour le transfert d'un acide nucléique in vivo comprenant l'administration d'une composition telle que décrite ci-avant. L'invention peut être utilisée par exemple chez l'animal, pour la création de modèles pathologiques, ou l'étude de la régulation de gènes, ou également chez l'homme, dans des études de marquage, de biodisponibilité, ou à des fins médicales.

Pour la régulation de l'expression, les compositions de l'invention comprennent avantageusement de la tétracycline ou tout analogue de tétracycline, capable d'agir sur l'activité du transactivateur tTA. Un tel analogue est constitué par exemple par la doxycycline (Kistner et al., PNAS 93 (1996) 10933). D'autres analogues utilisables dans la présente invention ont été décrits par exemple par Alvarez et al. (Gene Ther. 4 (1997) 993). Les doses d'analogues peuvent être aisément adaptées par l'homme du métier en fonction de l'analogue et des constructions utilisées. A titre indicatif, des doses de 0,1 ng/ml de doxycycline sont suffisantes in vitro pour bloquer totalement l'expression de l'acide nucléique dans les constructions de l'invention.

La présente invention est particulièrement adaptée au traitement (i.e., à la diminution partielle ou complète) de maladies neurodégénératives telles que notamment la maladie de Parkinson (MP). La MP est une maladie caractérisée par la perte progressive des neurones dopaminergiques dans la substance noire. Un traitement à la L-DOPA est effectué pour tenter de contrôler les symptomes de la maladie, mais son efficacité décroît avec la progression de la maladie. La présente demande offre une alternative avantageuse pour le traitement de cette pathologie, par l'introduction d'un gène (Tyrosine hydroxylase, TH), impliqué dans la synthèse de dopamine in vivo. Les expériences réalisées par traitement pharmacologique à long terme avec des composés dopaminergiques ont montré que ces traitements induisent un effet non régulé généralisé, conduisant dans la plupart des cas à des fluctuations très gênantes de la réponse des neurones moteurs. La présente invention est particulièrement adaptée à ce type de situation puisqu'elle permet un contrôle fin et local de la synthèse de L-DOPA, qui sont essentiels à l'obtention d'une réponse moteur reproduisant physiologiquement les mécanismes complexes de la stimulation dopaminergique.

La présente demande sera décrite plus en détails à l'aide des exemples qui suivent, qui doivent être considérés comme illustratifs et non limitatifs.

### LEGENDE DES FIGURES

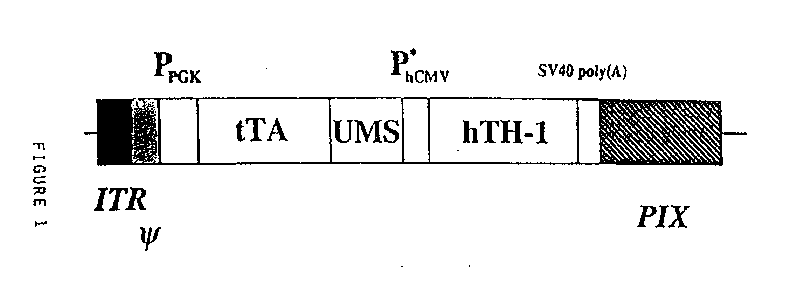

Figure 1 : Représentation de la structure du génome d'un adénovirus comprenant un acide nucléique selon l'invention. Les séquences terminales répétées inversées (ITR) et la séquence d'encapsidation (ψ) sont indiquées. pIX représente la séquence codant pour la protéine adénovirale IX.
Figure 2 : Panneau (a) : Infection de progéniteur nerveux humain avec l'adénovirus AdPGK.tet.hTH-1 à différentes MOls. Les valeurs indiquées sont des moyennes de quatre expériences.
   Panneaux (b) et (c) : Production in vitro de TH dans des progéniteurs nerveux humains, mesurée par immunocytochimie sept jours après infection MOI = 140. Panneau (b) sans traitement, panneau (c) après traitement en présence de doxycycline (10ng/ml) immédiatement après infection et jusqu'au jour 7. Les agrandissements sont 360 (panneau (b)) et 220 (panneau (c)).

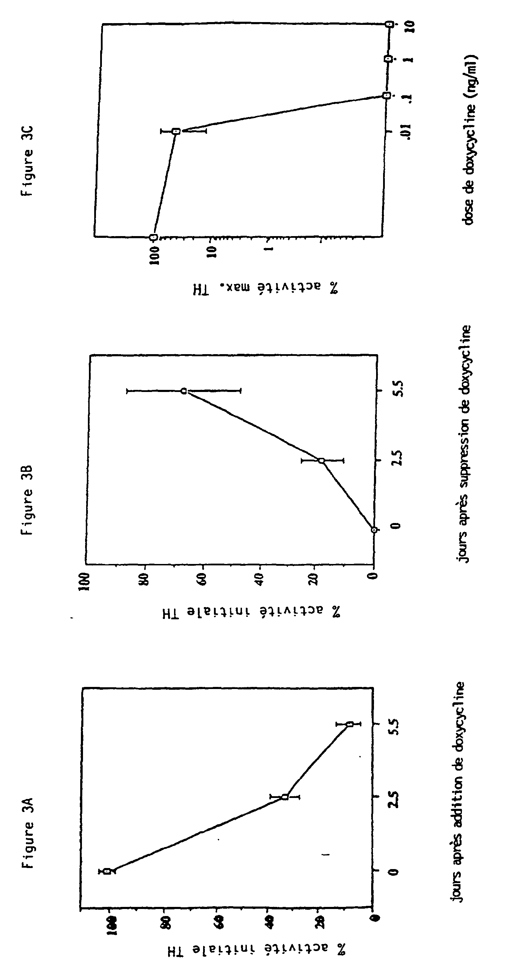

Figure 3 : Cinétique de la disparition (a) et de la reprise (b), de l'activité hTH dans des cultures infectées avec l'adénovirus AdPGK.tet.hTH1 (MOI = 40) après addition (a) ou retrait (b) de doxycycline (5ng/ml) au jour 0 (5,5 jours après infection). L'activité hTH dans les cultures non traitées au jour 0 a été déterminée comme 100%. (c) Effet de différentes doses de doxycycline sur l'expression de hTH déterminée au jour 11 après infection. Les valeurs sont des moyennes de 4 expériences au moins.

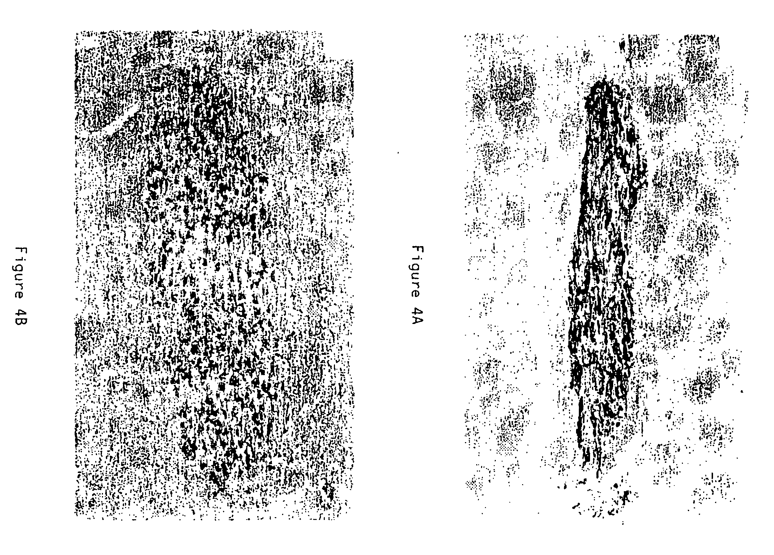

Figure 4 : Immunoréactivité à la TH dans les greffes intrastriatales de progéniteurs nerveux humains infectés avec l'adénovirus AdPGK.tet.hTH-1 (MOI = 40) mesurée par immunohistochimie anti-TH sur des sections cryostatées de cerveaux de 15µm (a) animaux non traités et (b) un animal traité quotidiennement avec la doxycycline. Le marquage violet (a) (b) révèle la présence de cellules humaines hybridant avec la sonde alu marqué à la digoxygénine consécutivement à l'incubation avec un anticorps anti-digoxygénine couplé à la phosphatase alcaline et coloration subséquente avec un substrat spécifique. Agrandissement 110.

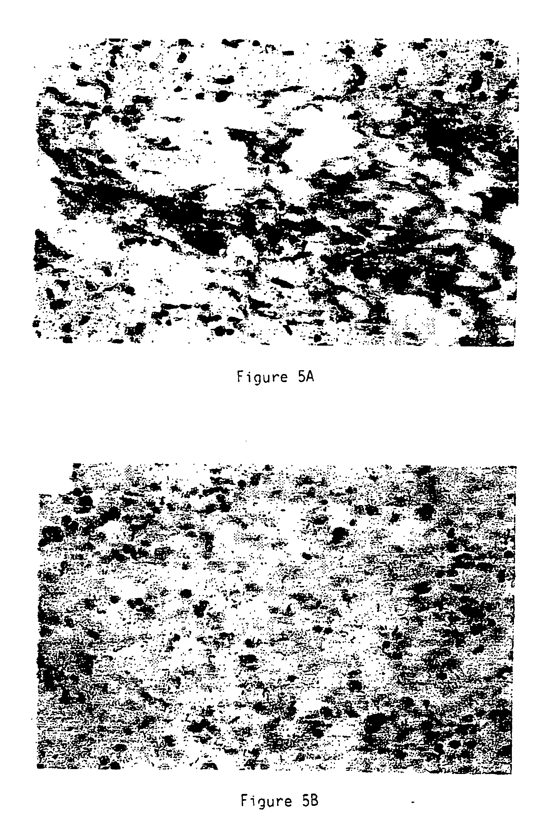

Figure 5 : Immunoréactivité TH dans les greffes de progéniteurs nerveux humains infectées avec l'adénovirus AdPGK.tet.hTH1 (MOI = 40) mesurée par immunohistochimie anti-TH et hybridation in situ en utilisant un oligonucléotide antisens spécifique de la HTH-1 marqué au souffre 35 sur des sections de cerveaux cryostatées de 15 µm de (a) un animal non traité et (b) un animal traité quotidiennement avec la doxycycline. Les coupes ont été contremarqués avec du violet cresyl. Agrandissement 450.
Figure 6 : Séquence nucléotidique du gène tTA (A) ; de la séquence UMS (B) ; de la séquence Op (C) et du promoteur minimal CMV (D).

### EXEMPLES

### 1 - Matériels et Méthodes

### 1. Construction de l'adénovirus AdPGK. Tet. HTH-1.

Un fragment d'ADN comprenant la séquence codant pour le transactivateur du système de régulation par la tétracycline (tTA) et pour le promoteur minimum du cytomégalovirus humain (PhCMV) a été excisé à partir du plasmide pUMS-luc (Corti et al. Neuroreport 7 (1996) 1655) par digestion avec les enzymes XhoI et ScaI et inséré dans le plasmide navette pCMVluclX (fourni par M.C. Geoffroy) digéré par les enzymes BgIII/Clal. Le vecteur ptetlX a ainsi été obtenu. Un fragment BgIII/HindIII contenant le cDNA de la tyrosine hydroxylase I humaine (hTH-1) (Grima B. et al. Nature 1987, 326:707-711) suivi du signal de polyadenylation du virus SV40 a été inséré entre les sites Clal et EcoRV du vecteur ptetIX, générant ainsi le vecteur ptetIXhTH-1. Le promoteur précoce immédiat du cytomégalovirus humain (phCMV) contrôlant l'expression de tTA dans le vecteur ptetIXhTH a été remplacé par le promoteur ubiquitaire murin du gène de la phosphoglycérate kinase (PGK) (Adra et al., Gene 1987; 60-65). Un fragment Xhol/Spll, contenant le promoteur PGK a été ligaturé entre les sites Spel et Spll du vecteur ptetlXhTH-1 pour générer le plasmide pPGKtetlXhTH-1. Pour les recombinaisons homologues (Stratford et al. J. Clin. Invest. (1992) 626) le vecteur pPGKtetlXh TH-1 a été linéarisé avec Xmnl et cotransfecté avec le grand fragment Clal de l'adénovirus Adβgal dans la lignée 293 transcomplémentant la région E1. L'adénovirus recombinant AdPGK.tet.hTH-1 a été purifié deux fois par essai sur plaque. Un stock viral a été obtenu par amplification sur les cellules 293, purification sur gradient et chlorure de Césium puis sur colonne Sephadex. Le titre viral déterminé par titration sur plaque sur des cellules 293 est de 3,5x10¹⁰ pfu/ml.

### 2. Génération et culture de cellules progéniteurs nerveuses humaines.

Des cultures primaires de cerveaux embryonnaires humains ont été obtenues et propagées in vitro dans un milieu sans sérum supplémenté par du FGF basique comme décrit précédemment (Buc et al., Neurobiol. 10 (1995) 37).

### 3. Infection des cellules progéniteurs nerveuses humaines.

Pour les études in vitro, les cellules ont été ensemencées sur des plaques de culture de tissus à 12 puits à une densité de 6 à 7x10⁵ cellules par puits et incubées avec le virus à la multiplicité d'infections (MOI) choisie dans 400 µl d'un milieu défini dépourvu de sérum (DS-FM). Après 6 heures les surnagenants sont éliminés et remplacés par un volume équivalent de DS-FM. A différents temps après l'infection, les cellules sont récoltées et centrifugées (4000 RPM, 5 min) et les culots sont congelés à -80°C pour les essais enzymatiques (Reinhardt et al., life sciences, (1986) 21-85).

Pour les transplantations, les cellules ont été ensemencées sur des plaques de cultures de tissus de 6 cm de diamètre à une densité de 5x10⁶ cellules par plaque et incubées avec le virus AdPGK.tet.hTH-1 à une MOI de 40 pfu par cellule pendant 6 heures. Le jour après l'infection, les cellules ont été récoltées comme décrit précédemment (Sabaté et al., Nature Genetics, 9 (1995) 256), resuspendues dans du milieu DS-FM a une densité de 4x10⁵ cellules par µl et conservées sur de la glace pendant toutes les opérations de greffes.

### 4. Lésions et greffes intracérébrales.

Cinq à six semaines avant les greffes intracérébrales, de la 6-hydroxydopamine (6-OHDA) a été injectée de manière stéréotaxique dans le trajet dopaminergique mesostriatal gauche ascendant de rat adulte Sprague-Dawley (Chaarles-River), comme décrit précédemment (Horellou et al., Neuron 5 (1990), 393). Seize rats ont été implantés sous anesthésie avec 800µl par kg d'un mélange dans un rapport 1:1 de ketamine (UVA) et de Rompun (Bayer). Un µl de suspension cellulaire a été injecté en utilisant une seringue Hamilton de 10 µl dans chacun des deux sites du striatum lésé aux coordonnées stéréotaxiques suivantes : + 0,7 antérieur de la bregma (AB), + 2,5 latéral de la ligne centrale (LM), 5 ventral sur la surface durale (V) et +1,5 AB, -2,5 LM, 5V (barre de départ fixée à 0). Les animaux ont été injectés quotidiennement avec 10 mg par kg de cyclosporine.

### 5. Hybridation in situ et immunohystochimie

Quatre semaines après l'implantation, les animaux ont été perfusés comme décrits précédemment (Sabaté et al. précité). Les cerveaux ont été récupérés, fixés dans 4 % de pfa, conservés en milieu PBS, 20% de sucrose. Ultérieurement, des sections de 15 µm ont été obtenues.

Les cellules humaines ont été détectées par hybridation en utilisant une amorce alu spécifique de l'homme marquée avec des nucléotides taggés à la digoxigenine (5'-XTTgCAgTgAgCCgAgATCgCgCC-3'). Après une étape de dénaturation initiale (20 minutes dans 95% de formamide, 0,1X SSC à 75°, sous agitation), des coupes ont été traitées comme décrit précédemment (Dumas et al., J. Chem. Neuroanat. 5 (1992) 11). L' ARN de la TH-1 humaine a été détecté dans les greffes par hybridation in situ avec un oligonucléotide marqué au souffre 35 dirigé contre l'exon 1 de la TH humaine (5'-TgCCTgCTTggCgTCCAgCTCAgACA-3'). Les conditions d'hybridation sont identiques à celles décrites par Lanièce et al. (J. Neurochem. 66 (1996) 1819). Pour l'immunohistochimie, les coupes ont été traitées selon les techniques standard.

### Il - Résultats

### 1. Génération d'un adénovirus codant la TH-1 humaine sous le contrôle d'un système de régulation selon l'invention (AdPGK.tet.hTH-1).

Pour obtenir une expression régulée du transgène hTH-1 à partir d'un seul vecteur adénoviral, le système de régulation négatif à la tétracycline tet-off initialement basé sur l'utilisation de deux vecteurs a été modifié. Le transactivateur tTA sensible à la tétracycline et l'ADNc de la TH-1 humaine ont été insérés dans un squelette d'adénovirus Ad5 délété pour les régions E1 et E3. Dans le vecteur AdPGK.tet.hTH-1, l'expression de tTA est sous le contrôle du promoteur ubiquitaire du gène murin de la phosphoglycérate kinase (PGK), alors que la transcription de la TH humaine est contrôlée par un promoteur minimun sensible au tTA (le promoteur CMV). La séquence UMS a été insérée entre le tTA et le promoteur CMV pour limiter les interférences transcriptionnelles entre les deux unités de transcription.

### 2. Infection de progéniteurs nerveux humain avec AdPGK.tet.hTH-1 : expression régulée in vitro.

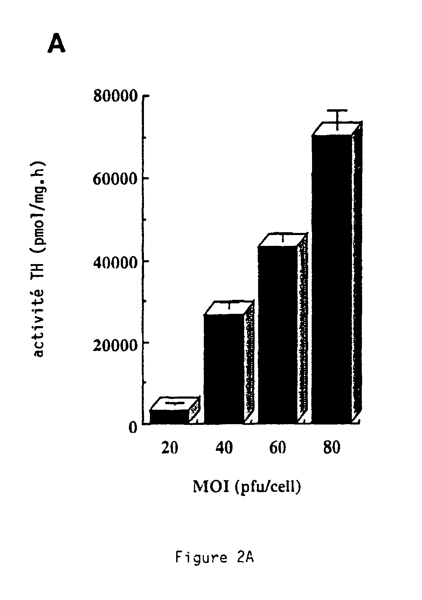

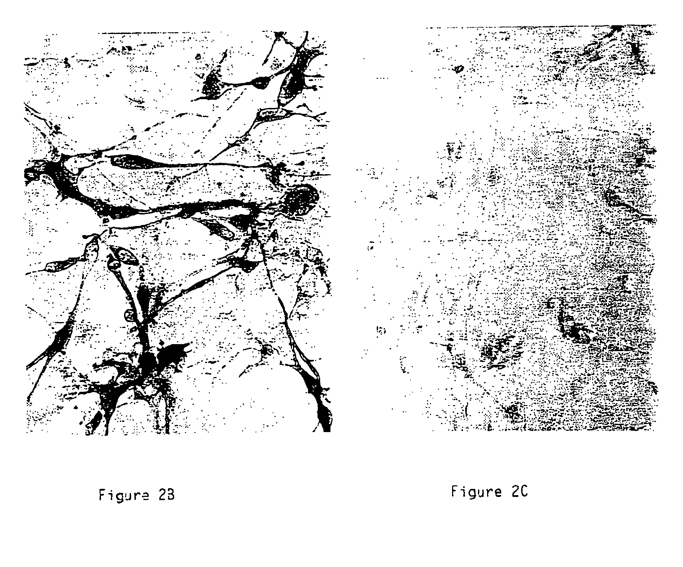

Pour déterminer si l'adénovirus AdPGK.tet.hTH-1 pouvait conduire à une synthèse de TH active dans les progéniteurs nerveux humains, des cellules ont été infectées à différentes multiplicités d'infection (figure 2a). Les résultats obtenus montrent que l'activité TH dans les cellules infectées augmente en fonction de la dose de vecteurs utilisée ce qui démontre que l'adénovirus AdPGK.tet.hTH-1 est fonctionnel.

La possibilité de contrôler l'expression du gène hTH-1 avec la doxycycline a été étudiée dans les cellules infectées par l'adénovirus. Lorsque l'antibiotique a été ajouté (10ng/ml) au milieu de culture immédiatement après l'infection, aucune activité TH n'a pu être mise en évidence à aucun moment, même à la plus forte dose de virus. De plus, aucune cellule présentant une immunoréactivité TH n'a été identifiée (figure 2b et 2c).

La possibilité que l'expression du gène puisse également être arrêtée ultérieurement dans les cellules exprimant déjà le transgène a ensuite été testée. Pour cela la doxycycline a été ajoutée au milieu de culture 5,5 jours après l'infection; lorsque l'activité TH était déjà détectable dans les cellules. Les résultats obtenus montrent que l'activité enzymatique décroît progressivement et disparaît presque totalement 5,5 jours après l'addition de doxycycline (figure 3a). Ainsi, la transcription du gène TH-1 dans les conditions de l'invention dans des progéniteurs nerveux humains peut être régulée négativement de manière très efficace.

Pour tester s'il est possible d'induire à nouveau une activité du transgène, l'activité TH a été suivie après retrait de la doxycycline du milieu de culture des cellules infectées (figure 3d). Les résultats obtenus montrent que l'activité TH augmente progressivement dans les cellules ce qui indique que l'inhibition à la doxycycline du transgène est réversible.

Pour déterminer la dose minimum d'antibiotique requise pour inhiber l'expression du gène, les cellules ont ensuite été incubées avec des concentrations variables de doxycycline immédiatement après l'infection (figure 3c). Une concentration de 0,1 ng/ml est suffisante pour empêcher l'apparition de l'activité TH alors que la dose de 0,01 ng/ml une activité TH peut être détectée.

### 3. Transplantation dans le cerveau de progéniteurs nerveux humains infectés avec l'adénovirus AdPGKtet.hTH-1 contrôle de l'expression in vivo.

Cet exemple met en évidence la capacité du système de l'invention et notamment des cellules progéniteurs nerveuses humaines infectées par l'adénovirus de médier une expression de TH humaine in vivo, et également la capacité du système de l'invention de réguler cette expression in vivo notamment par la doxcycline. Les cellules ont été infectées avec l'adénovirus AdPGK.tet.hTH-1 puis implantées dans le striatum de rats ayant subi une lésion unilatérale de la voie dopaminergique provoquée par l'injection de la toxine 6-OHDA. Pour faciliter la prise de la greffe, un nombre important de cellules (8x10⁵) suspendu à haute densité (4x10⁵/µl) a été greffé à chaque animal. Après la greffe, les animaux ont été soit non traités (n = 8) ou traités (n = 8) quotidiennement avec la doxycycline (1mg/ml dans l'eau). Quatre semaines après la greffe, les rats ont été sacrifiés et les cerveaux analysés pour l'expression de la TH.

Tous les cerveaux testés contiennent des greffes viables de cellules humaines comme démontré par hybridation in situ avec une sonde oligonucléotidique dirigé contre la séquence alu répétée spécifique de l'homme (figure 4a). Aucune différence dans la taille ou dans l'aspect des greffes n'a été observée entre les animaux traités et les animaux non traités à la doxycycline.

Les greffes dans les animaux non traités présentent une immunoréactivité TH importante (figure 4a). Le nombre de cellules exprimant la TH dans les greffes a ainsi été estimé entre 5 et 10%. Un pourcentage similaire de cellules ayant été remis en culture après la greffe dans un milieu sans sérum pendant six jours présentent également une immunoréactivité TH. Ces résultats montrent clairement, l'expression du gène in vivo, dans les mêmes conditions que in vitro.

En revanche, aucune cellule immunoréactive à la TH n'a été détectée dans aucun des animaux greffés ayant reçu la doxycycline (figure 4b). De ce fait, la doxycycline inhibe efficacement l'apparition de quantité de TH détectable par immunohistochimie dans les greffes.

Pour établir si l'effet de la doxycycline reflète un contrôle étroit de l'expression génétique au niveau transcriptionnel, une hybridation in situ a été réalisée sur des coupes en utilisant un oligonucléotide spécifique de l'ARN messager de la TH-1. Les greffes dans les animaux non traités ont été marquées de manière très importante. Le marquage était co-localisé avec les cellules immunoréactives (figure 5a). En revanche, aucun marquage spécifique n'a été détecté dans les greffes des animaux traités (figure 5b).

En conclusion, il peut être dit que la doxycycline inhibe efficacement la transcription du transgène dans le système de l'invention dans un contexte adénoviral introduit par des progéniteurs nerveux humains greffés.

## Revendications

1. Acide nucléique **caractérisé en ce qu'**il comprend
a) une première région comprenant un acide nucléique codant pour le transactivateur du système de régulation par la tétracyline (tTA) sous contrôle d'un promoteur non-viral modéré, et
b) une deuxième région comprenant un acide nucléique d'intérêt sous contrôle d'un promoteur sensible au tTA,
et **en ce que** les deux régions a) et b) sont disposées dans la même orientation transcriptionnelle

2. Acide nucléique selon la revendication 1, **caractérisé en ce qu'**il comprend, en outre, une troisième région c), disposée entre les deux régions a) et b), limitant les interférences transcriptionnelles entre les régions a) et b).

3. Acide nucléique selon la revendication 2, **caractérisé en ce que** la région c) comprend un terminateur transcriptionnel.

4. Acide nucléique selon la revendication 3, **caractérisé en ce que** le terminateur transcriptionnel est une séquence UMS.

5. Acide nucléique selon l'une quelconque des revendications précédentes, **caractérisé en ce que**, dans la région a), le promoteur modéré est un promoteur cellulaire.

6. Acide nucléique selon la revendication 5, **caractérisé en ce que** le promoteur modéré est un promoteur constitutif au tissu spécifique.

7. Acide nucléique selon la revendication 5, **caractérisé en ce que** le promoteur cellulaire modéré est choisi parmi le promoteur des gènes PGK, DHFR, EF1α, β-actine, β-globine et MHCα.

8. Acide nucléique selon l'une quelconque des revendications précédentes, **caractérisé en ce que** dans la région b), l'acide nucléique d'intérêt est un acide nucléique codant pour une protéine ou un polypeptide d'intérêt.

9. Acide nucléique selon la revendication 8, **caractérisé en ce que** la protéine ou le polypeptide d'intérêt sont choisis parmi les neurotransmetteurs, leurs précurseurs ou leurs enzymes de synthèse et les facteurs neurotrophiques.

10. Acide nucléique selon l'une quelconque des revendications précédentes, **caractérisé en ce que**, dans la région b), le promoteur est un promoteur fonctionnel dans les cellules de mammifère.

11. Acide nucléique selon la revendication 3 **caractérisé en ce qu'**il comprend :
a) une première région comprenant un acide nucléique codant pour le transactivateur du système de régulation par la tétracycline (tTA) sous contrôle du promoteur du gène PGK, et,
b) une deuxième région comprenant un acide nucléique codant pour la tyrosine hydroxylase humaine sous contrôle d'un promoteur CMV minimal, modifié de façon à comporter 1 à 10 séquences tetOp,
c) une troisième région comprenant la séquence UMS,
et **en ce que** les deux régions a) et b) sont disposées dans la même orientation transcriptionnelle.

12. Vecteur comprenant un acide nucléique selon l'une quelconque des revendications 1 à 11.

13. Vecteur selon la revendication 12 **caractérisé en ce qu'**il s'agit d'un vecteur viral.

14. Vecteur selon la revendication 13 **caractérisé en ce que** le vecteur viral est en adénovirus.

15. Cellule nerveuse comprenant un acide nucléique selon l'une quelconque des revendications 1 à 11 ou un vecteur selon la revendication 12.

16. Cellule nerveuse selon la revendication 15 **caractérisée en ce qu'**il s'agit d'une cellule mammifère.

17. Cellule selon la revendication 16, **caracterisée en ce qu'**il s'agit d'une cellule humaine.

18. Cellule nerveuse génétiquement modifiée par un adénovirus recombinant comprenant un acide nucléique selon la revendication 11.

19. Composition comprenant des cellules selon rune des revendications 15 à 18.

20. Utilisation d'un acide nucléique selon l'une des revendications 1 à 11 ou d'un vecteur selon la revendication 12 ou d'une composition selon la revendication 19 pour la préparation d'une composition destinée au traitement des maladies neurodégénérative des traumatismes ayant endommagé le système nerveux ou des dégénérescences rétiniennes.

21. Utilisation selon la revendication 20 **caractérisé en ce que** la maladie dégénérative est la maladie de Parkinson.

## Patentansprüche

1. Nukleinsäure, **dadurch gekennzeichnet, dass** sie umfasst:
a) eine erste Region, umfassend eine Nukleinsäure, die für den Transaktivator des Tetracyclin-basierten Regulationssystems (tTA) codiert, unter Kontrolle eines moderaten nicht-viralen Promotors, und
b) eine zweite Region, umfassend eine Nukleinsäure von Interesse unter Kontrolle eines tTA-sensitiven Promotors,
und **dadurch gekennzeichnet, dass** die beiden Regionen a) und b) in der gleichen Transkriptionsrichtung angeordnet sind.

2. Nukleinsäure gemäß Anspruch 1, **dadurch gekennzeichnet, dass** sie außerdem eine dritte Region c) umfasst, die zwischen den beiden Regionen a) und b) angeordnet ist und die transkriptionellen Interferenzen zwischen den Regionen a) und b) beschränkt.

3. Nukleinsäure gemäß Anspruch 2, **dadurch gekennzeichnet, dass** die Region c) einen transkriptionellen Terminator umfasst.

4. Nukleinsäure gemäß Anspruch 3, **dadurch gekennzeichnet, dass** der transkriptionelle Terminator eine UMS-Sequenz ist.

5. Nukleinsäure gemäß einem der vorhergehenden Ansprüche, **dadurch gekennzeichnet, dass** in der Region a) der moderate Promotor ein zellulärer Promotor ist.

6. Nukleinsäure gemäß Anspruch 5, **dadurch gekennzeichnet, dass** der moderate Promotor ein konstitutiver oder Gewebe spezifischer Promotor ist.

7. Nukleinsäure gemäß Anspruch 5, **dadurch gekennzeichnet, dass** der moderate zelluläre Promotor aus dem Promotor der PGK-, DHFR-, EF1α-, β-Actin-, β-Globin- und MHCα-Gene ausgewählt ist.

8. Nukleinsäure gemäß einem der vorhergehenden Ansprüche, **dadurch gekennzeichnet, dass** in der Region b) die Nukleinsäure von Interesse eine Nukleinsäure ist, die für ein Protein oder Polypeptid von Interesse codiert.

9. Nukleinsäure gemäß Anspruch 8, **dadurch gekennzeichnet, dass** das Protein oder Polypeptid von Interesse aus den Neurotransmittern, ihren Vorläufern oder ihren Synthese-Enzymen und den neurotrophen Faktoren ausgewählt ist.

10. Nukleinsäure gemäß einem der vorhergehenden Ansprüche, **dadurch gekennzeichnet, dass** in der Region b) der Promotor ein in Säugerzellen funktioneller Promotor ist.

11. Nukleinsäure gemäß Anspruch 3, **dadurch gekennzeichnet, dass** sie umfasst:
a) eine erste Region, umfassend eine Nukleinsäure, die für den Transaktivator des Tetracyclin-basierten Regulationssystems (tTA) codiert, unter Kontrolle des Promotors des PGK-Gens, und
b) eine zweite Region, umfassend eine Nukleinsäure, die für die humane Tyrosin-Hydroxylase codiert, unter Kontrolle eines minimalen CMV-Promotors, der modifiziert ist, so dass er 1 bis 10 tetOp-Sequenzen umfasst,
c) eine dritte Region, umfassend die UMS-Sequenz,
und **dadurch gekennzeichnet, dass** die beiden Regionen a) und b) in der gleichen Transkriptionsrichtung angeordnet sind.

12. Vektor, umfassend eine Nukleinsäure gemäß einem der Ansprüche 1 bis 11.

13. Vektor gemäß Anspruch 12, **dadurch gekennzeichnet, dass** es sich um einen viralen Vektor handelt.

14. Vektor gemäß Anspruch 13, **dadurch gekennzeichnet, dass** der virale Vektor ein Adenovirus ist.

15. Nervenzelle, umfassend eine Nukleinsäure gemäß einem der Ansprüche 1 bis 11 oder einen Vektor gemäß Anspruch 12.

16. Nervenzelle gemäß Anspruch 15, **dadurch gekennzeichnet, dass** es sich um eine Säugerzelle handelt.

17. Zelle gemäß Anspruch 16, **dadurch gekennzeichnet, dass** es sich um eine humane Zelle handelt.

18. Nervenzelle, die mit einem rekombinanten Adenovirus, umfassend eine Nukleinsäure gemäß Anspruch 11, genetisch verändert ist.

19. Zusammensetzung, umfassend Zellen gemäß einem der Ansprüche 15 bis 18.

20. Verwendung einer Nukleinsäure gemäß einem der Ansprüche 1 bis 11 oder eines Vektors gemäß Anspruch 12 oder einer Zusammensetzung gemäß Anspruch 19 zur Herstellung einer Zusammensetzung für die Behandlung neurodegenerativer Krankheiten, das Nervensystem schädigender Verletzungen oder Netzhautdegenerationen.

21. Verwendung gemäß Anspruch 20, **dadurch gekennzeichnet, dass** die degenerative Krankheit die Parkinson Krankheit ist.

## Claims

1. A nucleic acid, wherein said nucleic acid comprises :
a) a first region comprising a nucleic acid encoding the transactivator of the tetracycline-regulated system (tTA) under the control of a moderate non-viral promoter; and
b) a second region comprising a nucleic acid of interest under the control of a tTA-sensitive promoter;
and wherein the two regions a) and b) are arranged in the same transcriptional orientation.

2. Nucleic acid according to claim 1, wherein said nucleic acid further comprises a third region c), which is arranged between the two regions a) and b), restricting the transcriptional interferences between regions a) and b).

3. Nucleic acid according to claim 2, wherein region c) comprises a transcription terminator.

4. Nucleic acid according to claim 3, wherein the transcription terminator is a UMS sequence.

5. Nucleic acid according any one of the preceding claims, wherein, in region a), the moderate promoter is a cellular promoter.

6. Nucleic acid according to claim 5, wherein the moderate promoter is a constitutive or tissue-specific promoter.

7. Nucleic acid according to claim 5, wherein the moderate cellular promoter is selected from the group consisting of the promoter of genes PGK, DHFR, EF1αa, β-actin, β-globin and MHCα.

8. Nucleic acid according any one of the preceding claims, wherein, in region b), the nucleic acid of interest is a nucleic acid encoding a protein or polypeptide of interest.

9. Nucleic acid according to claim 8, wherein the protein or polypeptide of interest is selected among neurotransmitters, their precursors, or the enzymes for synthesizing them, and neurotrophic factors.

10. Nucleic acid according any one of the preceding claims, wherein, in region b), the promoter is a promoter functional in mammalian cells.

11. Nucleic acid according to claim 3, wherein said nucleic acid comprises:
a) a first region comprising a nucleic acid encoding the transactivator of the tetracycline-regulated system (tTA) under the control of the promoter of the PGK gene;
b) a second region comprising a nucleic acid encoding human tyrosine hydroxylase under the control of a minimal CMV promoter modified so as to contain from 1 to 10 tetOp sequences;
c) a third region comprising a UMS sequence;
and wherein the two regions a) and b) are arranged in the same transcriptional orientation.

12. A vector comprising a nucleic acid according any one of claims 1 to 11.

13. Vector according claim 12, wherein said vector is a viral vector.

14. Vector according claim 13, wherein said viral vector is an adenovirus.

15. A nervous cell comprising a nucleic acid according any one of claims 1 to 11 or a vector according claim 12.

16. Nervous cell according claim 15, wherein the cell is a mammalian cell.

17. Cell according claim 16, wherein the cell is a human cell.

18. A nervous cell genetically modified by an adenovirus comprising a nucleic acid according to claim 11.

19. A composition comprising cells according any one of claims 15 to 18.

20. Use of a nucleic acid according any one of claims 1 to 11 or of a vector according claim 12 or of a composition according to claim 19 for the preparation of a composition for treating neurodegenerative diseases, traumas which have damaged the nervous system or retinal degenerations.

21. Use according to claim 20, wherein the neurodegenerative disease is the Parkinson's disease.
